# EUROPEAN PATENT SPECIFICATION

(11) **EP 2 974 745 B1**
(45) Date of publication and mention of the grant of the patent: **01.11.2023**
(21) Application number: 15176539.3
(22) Date of filing: 13.07.2015
(51) Int. Cl.: A61K 49/18

(54) **METHOD FOR IMMUNE CELL TRACKING**
VERFAHREN ZUR IMMUNZELLENVERFOLGUNG
PROCÉDÉ DE SUIVI DE CELLULES IMMUNITAIRES

(30) Priority: 14.07.2014 US 201462024225 P
(43) Date of publication of application: 20.01.2016
(73) Proprietor: MegaPro Biomedical Co., Ltd., Taipei 110 (TW)
(72) Inventor: CHEN, Chih-Lung, 407 Taichung City (TW); HSIEH, Wen-Yuan, 302 Zhubei City, Hsinchu County (TW); LIN, Chen-Hsuan, 900 Pingtung City, Pingtung City (TW); WANG, Shian-Jy, 310 Zhudong Township, Hsinchu County (TW)
(74) Representative: Cabinet Becker et Associés

(56) References cited:
- US-A1- 2012 329 129
- CHIH-LUNG CHEN ET AL: "A New Nano-sized Iron Oxide Particle with High Sensitivity for Cellular Magnetic Resonance Imaging", MOLECULAR IMAGING AND BIOLOGY, SPRINGER-VERLAG, NE, vol. 13, no. 5, 24 September 2010 (2010-09-24), pages 825-839, XP019956302, ISSN: 1860-2002, DOI: 10.1007/S11307-010-0430-X
- SOPHIE LAURENT ET AL: "Magnetic Iron Oxide Nanoparticles: Synthesis, Stabilization, Vectorization, Physicochemical Characterizations, and Biological Applications", CHEMICAL REVIEWS, vol. 108, no. 6, 1 June 2008 (2008-06-01), pages 2064-2110, XP055158397, ISSN: 0009-2665, DOI: 10.1021/cr068445e
- ROHRER MARTIN ET AL: "Comparison of magnetic properties of MRI contrast media solutions at different magnetic field strengths", INVESTIGATIVE RADIOLOGY, LIPPINCOTT WILLIAMS & WILKINS, US, vol. 40, no. 11, 1 November 2005 (2005-11-01), pages 715-724, XP009154777, ISSN: 0020-9996
- GHESQUIÈRE BART ET AL: "Metabolism of stromal and immune cells in health and disease", NATURE, NATURE PUBLISHING GROUP UK, LONDON, vol. 511, no. 7508, 9 July 2014 (2014-07-09), pages 167-176, XP037438218, ISSN: 0028-0836, DOI: 10.1038/NATURE13312 [retrieved on 2014-07-09]

## Description

### BACKGROUND

Immune cell tracking, which relates to monitoring immune cells' migration and accumulation, can effectively detect immune response such as immune rejection, a major cause of functional failure in patients who have received organ transplantation.

Immune response is generally monitored by periodically analyzing biopsy samples (e.g., an endomyocardial biopsy sample) to detect the presence of immune cell (e.g., T-cells and macrophages) in an organ associated with a disease.

This monitoring procedure has several drawbacks. First, as an invasive procedure, it tends to bring about adverse side effects. Further, it is prone to sampling errors that can yield false negative results. Moreover, it often fails to detect early acute or chronic rejection. Finally, it is an expensive procedure.

Immune cell tracking can also be achieved by administering to patients immune cells pre-labeled with magnetic nanoparticles. This process requires a tedious step of pre-labeling immune cells *ex vivo.*

Chih-Lung Chen et al., Molecular Imaging and Biology, vol. 13, no. 5, p. 825, report iron oxide nanoparticles with r2 relaxivity of 319.2 (mM.S)⁻¹.

There is a need to develop a simple and non-invasive method that is sensitive to track immune cells to detect early signs of disease.

### SUMMARY

Disclosed herein are magnetic nanoparticles for use in a simple and non-invasive method for tracking immune cells with biocompatible magnetic nanoparticles using magnetic resonance imaging ("MRI") scans. The method provides unexpectedly high sensitivity. The invention is as defined in the claims. The invention relates to biocompatible magnetic nanoparticles for use in a method of tracking immune cells as defined in the claims.

The method includes the following steps: (i) identifying a patient having a disease associated with an organ (e.g., heart, kidney, or lymph node); (ii) providing an aqueous suspension, free of particles greater than 1000 nm in size and containing biocompatible magnetic nanoparticles, (iii) administering the aqueous suspension into the blood stream of the patient; and (iv) subsequently obtaining a magnetic resonance image of the organ. Immune response is detected when the image shows the presence of hyperintense or hypointense spots (e.g., T2, T2*, or diffusion weighted MRI showing hypointense spots or T1 weighted MRI showing hyperintense spots). For example, the disease is cancer (e.g., lymphoma) or rejection of a transplanted organ (e.g., heart or kidney).

In one embodiment, the method is used to detect immune rejection, in which step (i) is to identify a patient having a transplanted organ and step (iv) is to obtain a T2-weighted magnetic resonance image of the transplanted organ. Immune rejection is then detected when the image shows the presence of hypointense spots.

The method described herein uses a contrast agent containing biocompatible magnetic nanoparticles to detect immune response with MRI technology.

The biocompatible magnetic nanoparticles each contain a superparamagnetic core that is covered by one or more biocompatible polymers, each of which has a polyethylene glycol group, a silane group, and a linker that links, via a covalent bond, the polyethylene glycol group and the silane group. Typically, these biocompatible magnetic nanoparticles each have a particle size of 10-1000 nm and a transverse magnetic relaxivity rate of 50-400. In one example, they each have a particle size of 15-200 nm and a transverse magnetic relaxivity rate of 120 to 400.

Generally, the superparamagnetic core contains an iron oxide, a cobalt oxide, a nickel oxide, or a combination thereof.

In each of the biocompatible polymers, which cover the superparamagnetic cores, the polyethylene glycol group typically has 5-1000 oxyethylene units (e.g., 10-200 oxyethylene units), and the silane group typically contains a C₁₋₁₀ alkylene group (e.g., a C₃-C₁₀ alkylene group).

The details of one or more embodiments are set forth in the description below.

### DETAILED DESCRIPTION

In the following detailed description, for purposes of explanation, numerous specific details are set forth in order to provide a thorough understanding of the disclosed embodiments.

The present invention relates to biocompatible magnetic nanoparticles for use in a method of tracking immune cells according to the claims.

The method of this invention is used to track immune cells using biocompatible magnetic nanoparticles, each of which contains a superparamagnetic core covered by one or more biocompatible polymers.

The biocompatible polymers are biodegradable and nontoxic to cells. Silane-containing biocompatible polymers, which can be easily functionalized as shown below, are suitable for preparation of biocompatible magnetic nanoparticles required by this method.

An exemplary biocompatible polymer has the following formula:

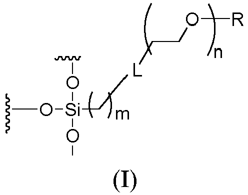

In formula (I), R is H, C₁-C₆ alkyl, C₂-C₆ alkenyl, C₂-C₆ alkynyl, C₃-C₁₀ cycloalkyl, C₁-C₁₀ heterocycloalkyl, aryl, heteroaryl, a C₁-C₁₀ carbonyl group, or a C₁-C₁₀ amine group; L is a linker; m is 1 to 10; and n is 5 to 1000.

A linker can be O, S, Si, C₁-C₆ alkylene, a carbonyl moiety containing two carbonyl groups and 2-20 carbon atoms, or a group having one of the following formula: 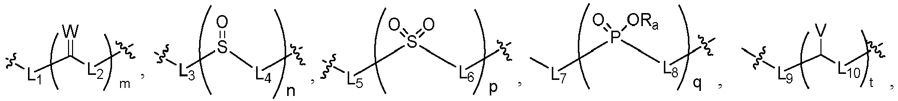 and 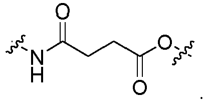 In these formula. each of m, n, p, and q, independently, is 1-6; W is O, S, or NR_{b}; each of L₁, L₃, L₅, L₇, and L₉, independently, is a bond, O, S, or NR_{c}; each of L₂, L₄, L₆, L₈, and L₁₀, independently, is a bond, O, S, or NR_{d}; and V is ORₑ, SR_{f}, or NR_{g}Rₕ, in which each of Rₐ, R_{b}, R_{c}, R_{d}, Rₑ, R_{f}, R_{g}, and Rₕ, independently, is H, OH, a C₁-C₁₀ oxyaliphatic radical, a C₁-C₁₀ monovalent aliphatic radical, a C₁-C₁₀ monovalent heteroaliphatic radical, a monovalent aryl radical, or a monovalent heteroaryl radical.

Another exemplary biocompatible polymer has the following formula:

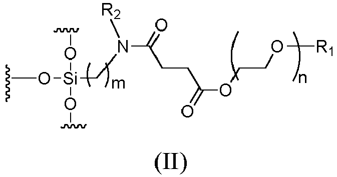

In formula (II), R₁ is H, C₁-C₆ alkyl, C₂-C₆ alkenyl, C₂-C₆ alkynyl, C₃-C₁₀ cycloalkyl, C₁-C₁₀ heterocycloalkyl, aryl, heteroaryl, a C₁-C₁₀ carbonyl group, or a C₁-C₁₀ amine group; R₂ is H, C₁-C₆ alkyl, C₂-C₆ alkenyl, C₂-C₆ alkynyl, C₃-C₁₀ cycloalkyl, C₁-C₁₀ heterocycloalkyl, aryl, or heteroaryl; m is 1 to 10 (e.g., 3-10); and n is 5 to 1000 (10-200). In a preferred embodiment, R₂ is H and the linker in formula (II) is

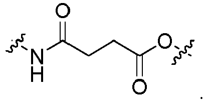

The term "aliphatic" herein refers to a saturated or unsaturated, linear or branched, acyclic, cyclic, or polycyclic hydrocarbon moiety. Examples include, but are not limited to, alkyl, alkylene, alkenyl, alkenylene, alkynyl, alkynylene, cycloalkyl, cycloalkylene, cycloalkenyl, cycloalkenylene, cycloalkynyl, and cycloalkynylene moieties. The term "alkyl" or "alkylene" refers to a saturated, linear or branched hydrocarbon moiety, such as methyl, methylene, ethyl, ethylene, propyl, propylene, butyl, butylenes, pentyl, pentylene, hexyl, hexylene, heptyl, heptylene, octyl, octylene, nonyl, nonylene, decyl, decylene, undecyl, undecylene, dodecyl, dodecylene, tridecyl, tridecylene, tetradecyl, tetradecylene, pentadecyl, pentadecylene, hexadecyl, hexadecylene, heptadecyl, heptadecylene, octadecyl, octadecylene, nonadecyl, nonadecylene, icosyl, icosylene, triacontyl, and triacotylene. The term "alkenyl" refers to a linear or branched hydrocarbon moiety that contains at least one double bond, such as -CH=CH-CH₃ and -CH=CH-CH₂-. The term "alkynyl" refers to a linear or branched hydrocarbon moiety that contains at least one triple bond, such as -C≡C-CH₃ and -C≡C-CH₂-. The term "cycloalkyl" refers to a saturated, cyclic hydrocarbon moiety, such as cyclohexyl and cyclohexylene.

The term "heteroaliphatic" herein refers to an aliphatic moiety containing at least one heteroatom (e.g., N, O, P, B, S, Si, Sb, Al, Sn, As, Se, and Ge). The term "heterocycloalkyl" refers to a cycloalkyl moiety containing at least one heteroatom. The term "oxyaliphatic" herein refers to an -O-aliphatic. Examples of oxyaliphatic include methoxy, ethoxy, n-propoxy, isopropoxy, n-butoxy, iso-butoxy, sec-butoxy, and tert-butoxy.

The term "aryl" herein refers to a C₆ monocyclic, C₁₀ bicyclic, C₁₄ tricyclic, C₂₀ tetracyclic, or C₂₄ pentacyclic aromatic ring system. Examples of aryl groups include, but are not limited to, phenyl, phenylene, naphthyl, naphthylene, anthracenyl, anthrcenylene, pyrenyl, and pyrenylene. The term "heteroaryl" herein refers to an aromatic 5-8 membered monocyclic, 8-12 membered bicyclic, 11-14 membered tricyclic, and 15-20 membered tetracyclic ring system having one or more heteroatoms (such as O, N, S, or Se). Examples of a heteroaryl group include, but are not limited to, furyl, furylene, fluorenyl, fluorenylene, pyrrolyl, pyrrolylene, thienyl, thienylene, oxazolyl, oxazolylene, imidazolyl, imidazolylene, benzimidazolyl, benzimidazolylene, thiazolyl, thiazolylene, pyridyl, pyridylene, pyrimidinyl, pyrimidinylene, quinazolinyl, quinazolinylene, quinolinyl, quinolinylene, isoquinolyl, isoquinolylene, indolyl, and indolylene.

Unless specified otherwise, aliphatic, heteroaliphatic, oxyaliphatic, alkyl, alkylene, alkenyl, alkynyl, cycloalkyl, heterocycloalkyl, aryl, and heteroaryl mentioned herein include both substituted and unsubstituted moieties. Possible substituents on cycloalkyl, heterocycloalkyl, aryl, and heteroaryl include, but are not limited to, C₁-C₁₀ alkyl, C₂-C₁₀ alkenyl, C₂-C₁₀ alkynyl, C₃-C₂₀ cycloalkyl, C₃-C₂₀ cycloalkenyl, C₃-C₂₀ heterocycloalkyl, C₃-C₂₀ heterocycloalkenyl, C₁-C₁₀ alkoxy, aryl, aryloxy, heteroaryl, heteroaryloxy, amino, C₁-C₁₀ alkylamino, C₂-C₂₀ dialkylamino, arylamino, diarylamino, C₁-C₁₀ alkylsulfonamino, arylsulfonamino, C₁-C₁₀ alkylimino, arylimino, C₁-C₁₀ alkylsulfonimino, arylsulfonimino, hydroxyl, halo, thio, C₁-C₁₀ alkylthio, arylthio, C₁-C₁₀ alkylsulfonyl, arylsulfonyl, acylamino, aminoacyl, aminothioacyl, amido, amidino, guanidine, ureido, thioureido, cyano, nitro, nitroso, azido, acyl, thioacyl, acyloxy, carboxyl, and carboxylic ester. On the other hand, possible substituents on aliphatic, heteroaliphatic, oxyaliphatic, alkyl, alkylene, alkenyl, and alkynyl include all of the above-recited substituents except C₁-C₁₀ alkyl. Cycloalkyl, heterocycloalkyl, aryl, and heteroaryl can also be fused with each other.

The biocompatible polymers described above include the polymers themselves, as well as their salts and solvates, if applicable. A salt, for example, can be formed between an anion and a positively charged group (e.g., amino) on a polymer. Suitable anions include chloride, bromide, iodide, sulfate, nitrate, phosphate, citrate, methanesulfonate, trifluoroacetate, acetate, malate, tosylate, tartrate, fumurate, glutamate, glucuronate, lactate, glutarate, and maleate. Likewise, a salt can also be formed between a cation and a negatively charged group (e.g., carboxylate) on a polymer. Suitable cations include sodium ion, potassium ion, magnesium ion, calcium ion, and an ammonium cation such as tetramethylammonium ion. The polymers also include those salts containing quaternary nitrogen atoms. A solvate refers to a complex formed between a polymer and a pharmaceutically acceptable solvent. Examples of a pharmaceutically acceptable solvent include water, ethanol, isopropanol, ethyl acetate, acetic acid, and ethanolamine.

Scheme (I) below shows a process of preparing an exemplary silane-containing biocompatible polymer.

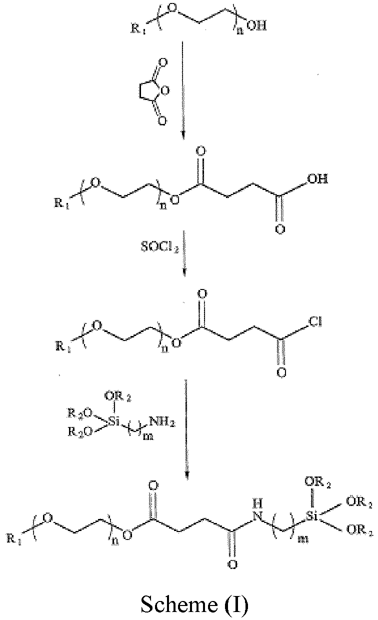

As shown in Scheme (I), alkoxyl-polyethylene glycol (molecular weight 2000) reacts with succinic anhydride in the presence of a base (e.g., dimethylaminopyridine) to form mPEG-COOH, which is subsequently converted to mPEG-COCl using thionyl chloride. Mixing mPEG-COCl with (3-aminopropyl)-triethoxysilane yields mPEG-silane.

A skilled person in the art can modify the process shown in Scheme (I) above to prepare biocompatible polymers using well-known methods. See R. Larock, Comprehensive Organic Transformations (VCH Publishers 1989); T. W. Greene and P. G. M. Wuts, Protective Groups in Organic Synthesis (3rd Ed., John Wiley and Sons 1999); L. Fieser and M. Fieser, Fieser and Fieser's Reagents for Organic Synthesis (John Wiley and Sons 1994); and L. Paquette, ed., Encyclopedia of Reagents for Organic Synthesis (John Wiley and Sons 1995) and subsequent editions thereof. Specific routes that can be used to synthesize the biocompatible polymers can be found in: (a) Rist et al., Molecules, 2005, 10, 1169-1178, (b) Koheler et al., JACS, 2004, 126, 7206-7211; and (c) Zhang et al., Biom. Mircod., 2004, 6:1 33-40.

The biocompatible polymers described above each can be coated onto a superparamagnetic core (e.g. iron-oxide nanoparticles) via covalent bonding to form a biocompatible magnetic nanoparticle for use in a contrast agent. The superparamagnetic core has a particle size of 8 to 25 nm (e.g., 12 to 25 nm and 15 to 20 nm) and an r2 relaxivity of 120 to 250 (mM·S)⁻¹ (e.g., 150 to 230 (mM·S)⁻¹ and 170 to 210 (mM·S)⁻¹). Preparation of a superparamagnetic core is well known in the art. See Laurent et al., Chem. Rev., 2008, 108, 2064-2110.

Described below is a typical procedure to prepare superparamagnetic nanoparticles. First, iron oxide nanoparticles are suspended in toluene, followed by stirring it with mPEG-silane at room temperature for 24 hours. The resultant biocompatible magnetic nanoparticles are hydrophilic and can be extracted to a water phase and subsequently purified by ultrafiltration. The biocompatible magnetic nanoparticles thus prepared each have an r2 relaxivity of 120 to 250 (mM·S)⁻¹ (e.g., 150 to 230 (mM·S)⁻¹ and 170 to 210 (mM·S)⁻¹).

The above-described biocompatible magnetic nanoparticle can be formulated into a contrast agent, which can be administered orally. Examples of a contrast agent include emulsions, aqueous suspensions, dispersions, and solutions. If desired, certain sweetening, flavoring, or coloring agents can be added.

The biocompatible magnetic nanoparticles can be administered into patients to label immune cells (*in vivo*), as described in examples below. Unlike administration of immune cells pre-labeled with nanoparticles (*in vivo*), administration of biocompatible magnetic nanoparticles in the absence of immune cells clearly has the advantages of fewer operative steps and fewer regulatory hurdles.

Not to be bounded by any theory, the biocompatible magnetic nanoparticles, once administered to a transplant patient, is taken up by immune cells (e.g., macrophages), which are accumulated at the organ when immune response occurs. In other words, the immune cells thus labeled can be readily monitored by T1, T2, T2*, or diffusion weighted MRI, shown as hyperintense spots in a T1 weighted MRI image or shown as hypointense spots in a T2, T2*, or diffusion weighted MRI image. A procedure of conducting T1, T2*, or diffusion weighted MRI is similar to that of conducting T2 weighted MRI reported in Mol. Imaging Biol., 2011, 13(5), 825-839.

The biocompatible magnetic nanoparticles described above, when administered to patients, exhibit unexpectedly high sensitivity to MRI for tracking immune cells to monitor immune response.

The specific examples below are to be construed as merely illustrative, Without further elaboration, it is believed that one skilled in the art can, based on the description herein, utilize the present embodiments to their fullest extent.

### Preparation of Biocompatible Iron Oxide Nanoparticles

Two biocompatible iron oxide nanoparticles of these embodiments were prepared following the procedure described below.

### Preparation of an iron oxide core

A mixture of FeCl₂·4H₂O (11.6 g; 0.058 mole), FeCl₃·6H₂O (11.6 g; 0.096 mole), and water (400 mL) was stirred at 300 rpm in a three-necked flask at 25°C.A sodium hydroxide solution (2.5 N; 170 mL) was added to the flask at a rate of 47 µl/sec, resulting a pH value of 11-12. Subsequently, oleic acid (20 mL) was added and stirred for 30 minutes, followed by addition of a 6 N HCl solution to adjust the pH value to about 1. The iron oxide core thus precipitated out of the mixture was collected by filtration, and washed with water for 4-5 times to remove excess oleic acid. It was then dried under vacuum to be used for coupling, as described below, with a biocompatible polymer.

### Preparation of biocompatible polymer mPEG-silane-750 and mPEG-silane-2000

The biocompatible polymer mPEG-silane-750 was prepared following the procedure described below.

A mixture of 300 g (0.4 moles) of methoxy-PEG (mPEG, molecular weight 750), succinic anhydride (48 g; 0.48 moles) and 4-dimethylamino-pyridine (DMAP; 19.5 g; 0.159 moles) were allowed to sit in a 1000-mL round bottom flask under vacuum (20 Torrs) for 2 hours. 600 mL of toluene was added to the mixture, which was then stirred at 30°C for one day to form mPEG-COOH.

Subsequently, 36 mL (0.48 moles) of thionyl chloride was added at a rate of 1 mL/min and the mixture was stirred for 2-3 hours. Thereafter, 333.8 mL (2.4 moles) of triethylamine was added at a rate of 1 mL/min to obtain pH around 6~7. After cooling to room temperature, the mixture containing mPEG-COCl was reacted with 94.5 mL (0.4 moles) of 3-aminopropyl triethoxysilane at room temperature for at least 8 hours to yield mPEG-silane-750.

mPEG-silane-750 was precipitated after 9 L of isopropyl ether was added to the reaction mixture. The solid product was collected by filtration, re-dissolved in 500 mL of toluene, and centrifuged at 5000 rpm for 5 minutes to collect a supernatant, to which was added 9 L of isopropyl ether. Brown oily liquid was separated from the isopropyl ether and dried under vacuum to obtain the biocompatible polymer mPEG-silane-750.

The biocompatible polymer mPEG-silane-2000 was prepared following the same procedure described above using a mixture of 800 g (0.4 moles) of methoxy-PEG (mPEG, molecular weight 2000), succinic anhydride (48 g; 0.48 moles) and 4-dimethylamino-pyridine (DMAP; 19.5 g; 0.159 moles).

### Coupling each of mPEG-silane-750 and mPEG-silane-2000 with iron oxide core

Each of biocompatible polymer mPEG-silane-750 and mPEG-silane-2000 (250 g) thus obtained was suspended in 1-1.2 L of a toluene solution containing 10 g of the iron oxide core prepared as described above. The suspension was stirred for 24 hours, followed by addition of water (1.5 L) for extraction. The extracted aqueous solution was filtered with an ultra-filtration device, washed with water, and then concentrated to 100 mL to obtain a biocompatible iron oxide nanoparticle suspension. The iron oxide nanoparticle, regardless of whether it was prepared from mPEG-silane-750 or mPEG-silane-2000, is designated as iTrast.

### Characterization of Biocompatible Iron Oxide Nanoparticle (itrast)

Transmission electron microscopy (TEM) images of the biocompatible magnetic nanoparticle iTrast thus obtained were taken using a JEOL JEM-2100F FieldEmission Transmission Electron Microscopy. The images showed that iTrast had an iron oxide core of the dimension 10-12 nm.

The transverse relaxivity (r2) and longitudinal (r1) relaxivity were determined following the procedures described in US Application Publication 2012/0329129 and Mol Imaging Biol, Chen et al., 2011, 13, 825-839. iTrast was determined to have an r2 of 205.3 ± 2.3 (mM·s)⁻¹ and an r1 of 18.6 ± 0.5 (mM·s)⁻¹.

### Detecting Migration and Accumulation of Macrophages in Transplantation

Studies to track macrophages in transplanted organs were performed following the procedures described below.

### Heart transplantation in Rats

The operative procedure for using the heterotopic working-heart model is described in PNAS, 2006, 103(6): 1852-1857. Inbred Brown Norway (BN; RT1ⁿ) and Dark Agouti (DA; RT1^{a}) rats were obtained from Harlan Laboratories Inc. (Indianapolis, IN). Allogeneic transplantation between different strains of rats (DA >BN) resulted in rejection, whereas syngeneic transplantation between the same strains of rats (D A > D A or BN→BN) caused no rejection. The rejection grade of the heart grafts was determined histopathologically according to the guidelines described in J. Heart Lung Transplant. , 1998, 17, 754-760 and J. Heart Transplant., 1990, 9, 587-593.

One day after the heart transplantation, each rat was intravenously injected with 3 mg/kg iTrast nanoparticles. It was observed that macrophages were heterogeneously distributed in the acutely rejected rat heart. Unexpectedly, *in vivo* MRI, conducted at day 6 post operation, indicated that macrophages labeled with iTrast nanoparticles accumulated at the allograft heart.

Histopathology confirmed an epicardium-to-endocardium progression pattern. More specifically, as rejection progressed over time, macrophage infiltration spreaded toward the inner part of the myocardium.

H&E and Perl's iron staining was performed on tissues from heart grafts harvested after *in vivo* MRI. Histological and immunohistochemical analyses of the grafts showed that iron-containing cells depicted by Perl's iron staining correlated with macrophage lineage ED1⁺ cells. The iron-containing cells correlated with ED1⁺ macrophages in the areas with more aggressive immune cell infiltration and disrupted myocardial integrity as revealed by H&E staining.

### Kidney Transplantation in Pigs

Major Histocompatibility Complex (MHC)-mismatched pigs were treated with high-dose tacrolimus for 12 days. Kidney allografts (n = 5) were then transplanted into these pigs. As expected, at day 14, all isolated kidney allografts were rejected as the serum creatinine concentration doubled compared to that at day 0.

One day after the kidney transplantation, each pig was intravenously injected with 3 mg/kg or 6 mg/kg iTrast particles. Accumulated macrophages labeled by nano-sized iTrast in the rejected kidney were unexpectedly detected by *in vivo* MRI at days 3, 6, 9, 12, and 16.

Indeed, it was also found that iTrast at both 3 mg/kg and 6 mg/kg enhanced the hypointense spots around cortex at day 9 and day 6, respectively, compared with serum creatinine, indicating immune rejection of all isolated kidneys.

### Tracking Macrophages on Lymph node

iTrast was studied to detect morphology change of a lymph node according to procedure shown below.

A mouse melanoma metastasis model using B16-F10 cells was induced in the forepaw. iTrast (2, 4, and 6 mg Fe/Kg) was administered to the mice during the tumor development, and the animals were repeatedly evaluated by MRI T2, T2*, and diffusion weighted imaging (i.e., T2WI, T2*WI, DWI) after the iTrast administration.

It was found that the tumor stages significantly affected the labeling results. When iTrast was given intravenously at a dosage of 4 mg Fe/kg, a transient labeling was unexpectedly observed in the sentinel and subsequent lymph nodes of animals having an early-stage tumor. The degree of transient signals was weakened in these lymph nodes when the tumor reached a late stage. Histology confirmed that the early-stage tumor was non-metastatic while the late-stage tumor was metastatic.

### OTHER EMBODIMENTS

All of the features disclosed in this specification may be combined in any combination.

## Claims

1. Biocompatible magnetic nanoparticles for use in a method of tracking immune cells, the method comprising:
identifying a patient having a disease associated with an organ;
providing an aqueous suspension containing biocompatible magnetic nanoparticles, the aqueous suspension being free of particles having a size greater than 1000 nm, the biocompatible magnetic nanoparticles each containing a superparamagnetic core that is covered by one or more biocompatible polymers, each of which has a polyethylene glycol group, a silane group, and a linker linking, via a covalent bond, the polyethylene glycol group and the silane group;
administering the aqueous suspension into the blood stream of the patient; and
after the administration step, obtaining a magnetic resonance image of the organ,
wherein the organ is kidney or lymph node, the biocompatible magnetic nanoparticles each have an r2 relaxivity of 120 to 250 (mM.S)⁻¹, and the presence of hyperintense or hypointense spots in the magnetic resonance image indicates immune response in the patient.

2. The biocompatible magnetic nanoparticles for use according to claim 1, wherein the magnetic resonance image is a T2 or T2* weighted magnetic resonance image.

3. The biocompatible magnetic nanoparticles for use according to claim 1 or 2,
wherein the disease is cancer or rejection of a transplanted organ.

4. The biocompatible magnetic nanoparticles for use according to claim 3, wherein the transplanted organ is heart or kidney.

5. The biocompatible magnetic nanoparticles for use according to claim 3, wherein the cancer is lymphoma.

6. The biocompatible magnetic nanoparticles for use according to any of claims 1 to 5, wherein the superparamagnetic core contains an iron oxide, a cobalt oxide, a nickel oxide, or a combination thereof; the polyethylene glycol group has 5-1000 oxyethylene units; the silane group contains a C₁₋₁₀ alkylene group; and the linker is O, S, Si, C₁-C₆ alkylene, a carbonyl moiety containing two carbonyl groups and 2-20 carbon atoms, or a group having one of the following formula: 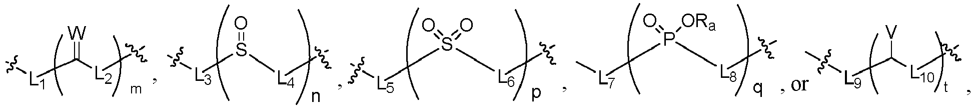 and 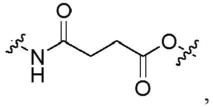 in which each of m, n, p, q, and t, independently, is 1-6; W is O, S, or NR_{b}; each of L₁, L₃, L₅, L₇, and L₉, independently, is a bond, O, S, or NR_{c}; each of L₂, L₄, L₆, L₈, and L₁₀, independently, is a bond, O, S, or NR_{d}; and V is ORₑ, SR_{f}, or NR_{g}Rₕ, each of Rₐ, R_{b}, R_{c}, R_{d}, Rₑ, R_{f}, R_{g}, and Rₕ, independently, being H, OH, a C₁-C₁₀ oxyaliphatic radical, a C₁-C₁₀ monovalent aliphatic radical, a C₁-C₁₀ monovalent heteroaliphatic radical, a monovalent aryl radical, or a monovalent heteroaryl radical.

7. The biocompatible magnetic nanoparticles for use according to any of claims 1 to 5, wherein the superparamagnetic core is a superparamagnetic iron oxide nanoparticle; the polyethylene glycol group has 10 to 200 oxyethylene units; the silane group contains C₃-C₁₀ alkylene; and the linker is a carbonyl moiety of the following formula:

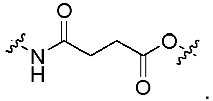

8. The biocompatible magnetic nanoparticles for use according to any of claims 1 to 5, wherein the superparamagnetic core is covered by one or more biocompatible polymers having the following formula: 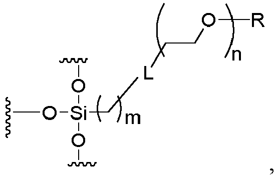 in which
R is H, C₁-C₆ alkyl, C₂-C₆ alkenyl, C₂-C₆ alkynyl, C₃-C₁₀ cycloalkyl, C₁-C₁₀ heterocycloalkyl, aryl, heteroaryl, a C₁-C₁₀ carbonyl group, or a C₁-C₁₀ amine group;
L is a linker;
m is 1 to 10; and
n is 5 to 1000.

9. The biocompatible magnetic nanoparticles for use according to claim 8, wherein the linker is O, S, Si, C₁-C₆ alkylene, a carbonyl moiety containing two carbonyl groups and 2-20 carbon atoms, or a group having one of the following formula: 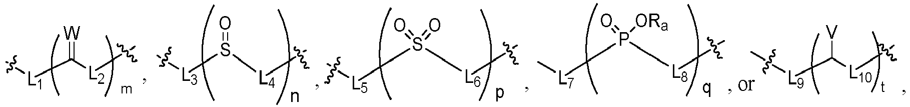 and 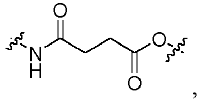 in which each of m, n, p, q, and t, independently, is 1-6; W is O, S, or NR_{b}; each of L₁, L₃, L₅, L₇, and L₉, independently, is a bond, O, S, or NR_{c}; each of L₂, L₄, L₆, L₈, and L₁₀, independently, is a bond, O, S, or NR_{d}; and V is ORₑ, SR_{f}, or NR_{g}Rₕ, each of Rₐ, R_{b}, R_{c}, R_{d}, Rₑ, R_{f}, R_{g}, and Rₕ, independently, being H, OH, a C₁-C₁₀ oxyaliphatic radical, a C₁-C₁₀ monovalent aliphatic radical, a C₁-C₁₀ monovalent heteroaliphatic radical, a monovalent aryl radical, or a monovalent heteroaryl radical.

10. The biocompatible magnetic nanoparticles for use according to any of claims 1 to 5, wherein the superparamagnetic core is covered by one or more biocompatible polymers having the following formula: 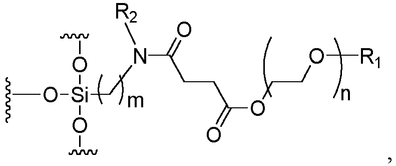 in which
R₁ is H, C₁-C₆ alkyl, C₂-C₆ alkenyl, C₂-C₆ alkynyl, C₃-C₁₀ cycloalkyl, C₁-C₁₀ heterocycloalkyl, aryl, heteroaryl, a C₁-C₁₀ carbonyl group, or a C₁-C₁₀ amine group;
R₂ is H, C₁-C₆ alkyl, C₂-C₆ alkenyl, C₂-C₆ alkynyl, C₃-C₁₀ cycloalkyl, C₁-C₁₀ heterocycloalkyl, aryl, or heteroaryl;
m is 1 to 10; and
n is 5 to 1000.

11. The biocompatible magnetic nanoparticles for use according to claim 10, wherein the biocompatible magnetic nanoparticles each have a particle size of 15-200 nm; the disease is rejection of a transplanted kidney; the superparamagnetic core is a superparamagnetic iron oxide nanoparticle; the polyethylene glycol group has 10 to 200 oxyethylene units; the silane group contains C₃-C₁₀ alkylene; the linker is a carbonyl moiety of the following formula: 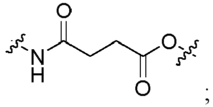 and the magnetic resonance image is a T2 or T2* weighted magnetic resonance image.

12. The biocompatible magnetic nanoparticles for use according to claim 10, wherein R₁ is H; R₂ is H, C₁-C₆ alkyl, a C₁-C₁₀ carbonyl group, or a C₁-C₁₀ amine group; m is 3 to 10; and n is 10 to 200.

13. The biocompatible magnetic nanoparticles for use according to any of claims 1-10, or 12, wherein the biocompatible magnetic nanoparticles each have a particle size of 10-1000 nm, preferably wherein the biocompatible magnetic nanoparticles each have a particle size of 15-200 nm.

## Patentansprüche

1. Biokompatible magnetische Nanopartikel zur Verwendung in einem Verfahren zur Verfolgung (tracking) von Immunzellen, wobei das Verfahren Folgendes umfasst:
Identifizieren eines Patienten mit einer mit einem Organ verbundenen Krankheit;
Bereitstellen einer wässrigen Suspension, die biokompatible magnetische Nanopartikel enthält, wobei die wässrige Suspension frei von Partikeln mit einer Größe von mehr als 1000 nm ist, wobei die biokompatiblen magnetischen Nanopartikel jeweils einen superparamagnetischen Kern enthalten, der von einem oder mehreren biokompatiblen Polymeren bedeckt ist, von denen jedes eine Polyethylenglykol-Gruppe, eine Silan-Gruppe und einen Linker aufweist, der über eine kovalente Bindung die Polyethylenglycol-Gruppe und die Silan-Gruppe verbindet;
Verabreichen der wässrigen Suspension in den Blutkreislauf des Patienten; und nach dem Verabreichungsschritt Anfertigung eines Magnetresonanzbildes des Organs,
wobei es sich bei dem Organ um eine Niere oder einen Lymphknoten handelt, die biokompatiblen magnetischen Nanopartikel jeweils eine r2-Relaxivität von 120 bis 250 (mM.S)⁻¹ aufweisen und das Vorhandensein hyperintenser oder hypointenser Flecken im Magnetresonanzbild auf eine Immunantwort des Patienten hinweist.

2. Biokompatible magnetische Nanopartikel zur Verwendung nach Anspruch 1, wobei das Magnetresonanzbild ein T2- oder T2*-gewichtetes Magnetresonanzbild ist.

3. Biokompatible magnetische Nanopartikel zur Verwendung nach Anspruch 1 oder 2, wobei die Krankheit Krebs oder eine Abstoßung eines transplantierten Organs ist.

4. Biokompatible magnetische Nanopartikel zur Verwendung nach Anspruch 3, wobei das transplantierte Organ Herz oder Niere ist.

5. Biokompatible magnetische Nanopartikel zur Verwendung nach Anspruch 3, wobei der Krebs ein Lymphom ist.

6. Biokompatible magnetische Nanopartikel zur Verwendung nach einem der Ansprüche 1 bis 5, wobei der superparamagnetische Kern ein Eisenoxid, ein Kobaltoxid, ein Nickeloxid oder eine Kombination davon enthält; die Polyethylenglykolgruppe hat 5-1000 Oxyethylen-Einheiten umfasst; die Silan-Gruppe eine C1-10-Alkylen-Gruppe enthält; und der Linker O, S, Si, C₁-C₆-Alkylen, eine Carbonyl-Einheit mit zwei Carbonyl-Gruppen und 2-20 Kohlenstoffatomen oder eine Gruppe mit einer der folgenden Formel ist: 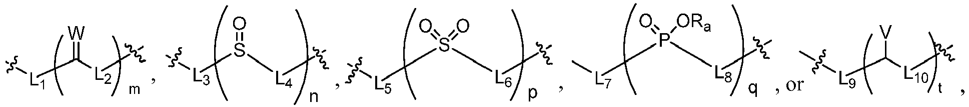 und 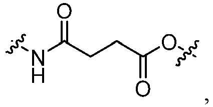 worin m, n, p, q und t jeweils unabhängig voneinander 1-6 sind; W O, S oder NR_{b} ist; L₁, L₃, L₅, L₇ und L₉ jeweils unabhängig voneinander eine Bindung, O, S oder NR_{c} ist; L₂, L₄, L₆, L₈ und L₁₀ jeweils unabhängig voneinander eine Bindung, O, S oder NR_{d} ist; und V ORₑ, SR_{f} oder NR_{g}Rₕ ist, wobei Rₐ, R_{b}, R_{c}, R_{d}, Rₑ, R_{f}, R_{g} und Rₕ jeweils unabhängig voneinander H, OH, ein oxyaliphatischer C₁-C₁₀-Rest oder ein monovalenter aliphatischer C₁-C₁₀-Rest, ein monovalenter heteroaliphatischer C₁-C₁₀-Rest, ein monovalenter Aryl-Rest oder ein monovalenter Heteroaryl-Rest sind.

7. Biokompatible magnetische Nanopartikel zur Verwendung nach einem der Ansprüche 1 bis 5, wobei der superparamagnetische Kern ein superparamagnetisches Eisenoxid-Nanopartikel ist; die Polyethylenglykol-Gruppe 10 bis 200 Oxyethylen-Einheiten aufweist; die Silan-Gruppe C₃-C₁₀-Alkylen enthält; und der Linker eine Carbonyl-Einheit der folgenden Formel ist:

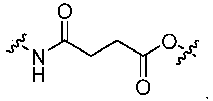

8. Biokompatible magnetische Nanopartikel zur Verwendung nach einem der Ansprüche 1 bis 5, wobei der superparamagnetische Kern von einem oder mehreren biokompatiblen Polymeren mit der folgenden Formel bedeckt ist: 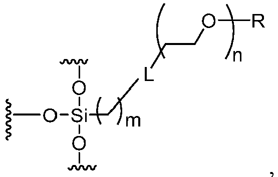 worin
RH, C₁-C₆ Alkyl, C₂-C₆ Alkenyl, C₂-C₆ Alkinyl, C₃-C₁₀ Cycloalkyl, C₁-C₁₀ Heterocycloalkyl, Aryl, Heteroaryl, eine C₁-C₁₀ Carbonyl-Gruppe oder eine C₁-C₁₀ Amin-Gruppe ist;
L ein Linker ist;
m 1 bis 10 ist; und
n 5 bis 1000 ist.

9. Biokompatible magnetische Nanopartikel zur Verwendung nach Anspruch 8, wobei der Linker O, S, Si, C₁-C₆-Alkylen, eine Carbonyleinheit mit zwei Carbonylgruppen und 2-20 Kohlenstoffatomen oder eine Gruppe mit einer der folgenden Formeln ist: 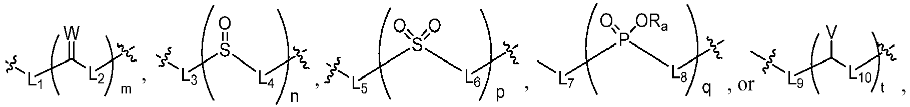 und 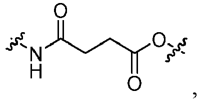 worin m, n, p, q und t jeweils unabhängig voneinander 1-6 sind; W O, S, oder NR_{b} ist; L₁, L₃, L₅, L₇, und L₉ sind jeweils unabhängig voneinander eine Bindungm O, S, oder NR_{c} ist; L₂, L₄, L₆, L₈, und L₁₀ jeweils unabhängig voneinander eine Bindung, O, S, oder NR_{d} ist; und V ORₑ, SR_{f}, oder NR_{g}Rₕ ist, wobei Rₐ, R_{b}, R_{c}, R_{d}, Rₑ, R_{f}, R_{g} und Rₕ jeweils unabhängig voneinander H, OH, ein oxyaliphatischer C₁-C₁₀-Rest, ein monovalenter aliphatischer C₁-C₁₀-Rest, ein monovalenter heteroaliphatischer C₁-C₁₀-Rest, ein monovalenter Aryl-Rest, oder ein monovalenter Heteroaryl-Rest.

10. Biokompatible magnetische Nanopartikel zur Verwendung nach einem der Ansprüche 1 bis 5, wobei der superparamagnetische Kern von einem oder mehreren biokompatiblen Polymeren mit der folgenden Formel bedeckt ist: 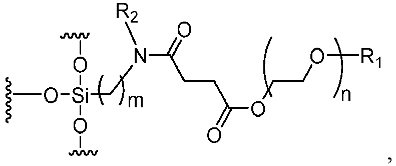 wobei
R₁ H, C₁-C₆ Alkyl, C₂-C₆ Alkenyl, C₂-C₆ Alkinyl, C₃-C₁₀ Cycloalkyl, C₁-C₁₀ Heterocycloalkyl, Aryl, Heteroaryl, eine C₁-C₁₀ Carbonyl-Gruppe, oder eine C₁-C₁₀ Amin-Gruppe ist;
R₂ H, C₁-C₆ Alkyl, C₂-C₆ Alkenyl, C₂-C₆ Alkinyl, C₃-C₁₀ Cycloalkyl, C₁-C₁₀ Heterocycloalkyl, Aryl, oder Heteroaryl ist;
m 1 bis 10 ist; und
n 5 bis 1000 ist.

11. Biokompatible magnetische Nanopartikel zur Verwendung nach Anspruch 10, wobei die biokompatiblen magnetischen Nanopartikel jeweils eine Partikelgröße von 15-200 nm haben; die Krankheit die Abstoßung einer transplantierten Niere ist; der superparamagnetische Kern ein superparamagnetisches Eisenoxid-Nanopartikel ist; die Polyethylenglykolgruppe 10 bis 200 Oxyethyleneinheiten aufweist; die Silangruppe C₃-C₁₀-Alkylen enthält; der Linker eine Carbonyleinheit der folgenden Formel ist: 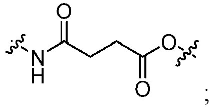 und das Magnetresonanzbild ist ein T2- oder T2*-gewichtetes Magnetresonanzbild.

12. Biokompatible magnetische Nanopartikel zur Verwendung nach Anspruch 10, wobei R₁ H ist; R₂ H, C₁-C₆ Alkyl, eine C₁-C₁₀ Carbonyl-Gruppe, oder eine C₁-C₁₀ Amin-Gruppe; m 3 bis 10 ist; und n 10 bis 200 ist.

13. Biokompatible magnetische Nanopartikel zur Verwendung nach einem der Ansprüche 1-10 oder 12, wobei die biokompatiblen magnetischen Nanopartikel jeweils eine Partikelgröße von 10-1000 nm aufweisen, vorzugsweise wobei die biokompatiblen magnetischen Nanopartikel jeweils eine Partikelgröße von 15-200 nm aufweisen.

## Revendications

1. Nanoparticules magnétiques biocompatibles pour une utilisation dans une méthode de suivi de cellules immunitaires, la méthode comprenant :
l'identification d'un patient ayant une maladie associée à un organe ;
la fourniture d'une suspension aqueuse contenant des nanoparticules magnétiques biocompatibles, la suspension aqueuse étant exempte de particules ayant une taille supérieure à 1000 nm, les nanoparticules magnétiques biocompatibles contenant chacune un coeur superparamagnétique qui est recouvert d'un ou plusieurs polymères biocompatibles, ayant chacun un groupe polyéthylèneglycol, un groupe silane, et un lieur liant, via une liaison covalente, le groupe polyéthylèneglycol et le groupe silane ;
l'administration de la suspension aqueuse dans la circulation sanguine du patient ;
et
après l'étape d'administration, l'obtention d'une image par résonance magnétique de l'organe,
l'organe étant un rein ou un ganglion lymphatique, les nanoparticules magnétiques biocompatibles ayant chacune une relaxivité r2 de 120 à 250 (mM.S)⁻¹, et la présence de points hyperintenses ou hypointenses dans l'image par résonance magnétique étant indicative d'une réponse immunitaire chez le patient.

2. Nanoparticules magnétiques biocompatibles pour une utilisation selon la revendication 1, l'image par résonance magnétique étant une image par résonance magnétique pondérée T2 ou T2*.

3. Nanoparticules magnétiques biocompatibles pour une utilisation selon la revendication 1 ou 2, la maladie étant un cancer ou un rejet de greffe d'organe.

4. Nanoparticules magnétiques biocompatibles pour une utilisation selon la revendication 3, l'organe greffé étant un coeur ou un rein.

5. Nanoparticules magnétiques biocompatibles pour une utilisation selon la revendication 3, le cancer étant un lymphome.

6. Nanoparticules magnétiques biocompatibles pour une utilisation selon l'une quelconque des revendications 1 à 5, le coeur superparamagnétique contenant un oxyde de fer, un oxyde de cobalt, un oxyde de nickel, ou une combinaison de ceux-ci ; le groupe polyéthylèneglycol ayant 5 à 1000 motifs oxyéthylène ; le groupe silane contenant un groupe alkylène en Ci à C₁₀ ; et le lieur est O, S, Si, un alkylène en C₁ à C₆, un fragment carbonyle contenant deux groupes carbonyle et 2 à 20 atomes de carbone, ou un groupe répondant à l'une des formules suivantes : 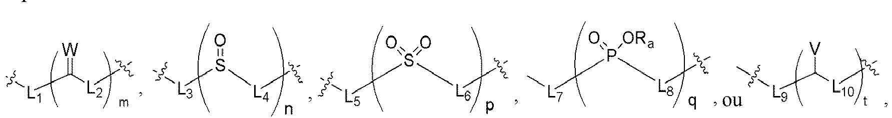 et 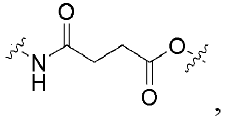 dans lesquelles chacun de m, n, p, q et t vaut indépendamment de 1 à 6 ; W est O, S ou NR_{b} ; chacun de L₁, L₃, L₅, L₇ et L₉ est indépendamment une liaison, O, S ou NR_{c} ; chacun de L₂, L₄, L₆, L₈ et L₁₀ est indépendamment une liaison, O, S ou NR_{d} ; et V est ORₑ, SR_{f} ou NR_{g}Rₕ, chacun de Rₐ, R_{b}, R_{c}, R_{d}, Rₑ, R_{f}, R_{g} et Rₕ étant indépendamment H, OH, un radical oxyaliphatique en C₁ à C₁₀, un radical aliphatique monovalent en C₁ à C₁₀, un radical hétéroaliphatique monovalent en C₁ à C₁₀, un radical aryle monovalent, ou un radical hétéroaryle monovalent.

7. Nanoparticules magnétiques biocompatibles pour une utilisation selon l'une quelconque des revendications 1 à 5, le coeur superparamagnétique étant une nanoparticule d'oxyde de fer superparamagnétique ; le groupe polyéthylèneglycol ayant 10 à 200 motifs oxyéthylène ; le groupe silane contenant un alkylène en C₃ à C₁₀ ; et le lieur étant un fragment répondant à la formule suivante :

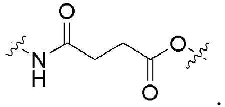

8. Nanoparticules magnétiques biocompatibles pour une utilisation selon l'une quelconque des revendications 1 à 5, le coeur superparamagnétique étant recouvert d'un ou plusieurs polymères biocompatibles répondant à la formule suivante : 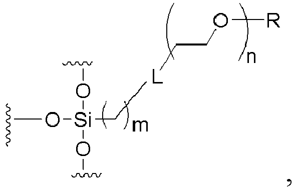 dans laquelle
R est H, un alkyle en C₁ à C₆, un alcényle en C₂ à C₆, un alcynyle en C₂ à C₆, un cycloalkyle en C₃ à C₁₀, un hétérocycloalkyle en C₁ à C₁₀, un aryle, un hétéroaryle, un groupe carbonyle en C₁ à C₁₀, ou un groupe amine en C₁ à C₁₀ ;
L est un lieur ;
m vaut de 1 à 10 ; et
n vaut de 5 à 1000.

9. Nanoparticules magnétiques biocompatibles pour une utilisation selon la revendication 8, le lieur étant O, S, Si, un alkylène en C₁ à C₆, un fragment carbonyle contenant deux groupes carbonyle et 2 à 20 atomes de carbone, ou un groupe répondant à l'une des formules suivantes : 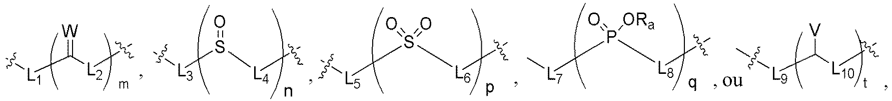 et 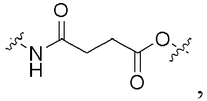 dans lesquelles chacun de m, n, p, q et t vaut indépendamment de 1 à 6 ; W est O, S ou NR_{b} ; chacun de L₁, L₃, L₅, L₇ et L₉ est indépendamment une liaison, O, S ou NR_{c} ; chacun de L₂, L₄, L₆, L₈ et L₁₀ est indépendamment une liaison, O, S ou NR_{d} ; et V est ORₑ, SR_{f} ou NR_{g}Rₕ, chacun de Rₐ, R_{b}, R_{c}, R_{d}, Rₑ, R_{f}, R_{g} et Rₕ étant indépendamment H, OH, un radical oxyaliphatique en C₁ à C₁₀, un radical aliphatique monovalent en C₁ à C₁₀, un radical hétéroaliphatique monovalent en C₁ à C₁₀, un radical aryle monovalent, ou un radical hétéroaryle monovalent.

10. Nanoparticules magnétiques biocompatibles pour une utilisation selon l'une quelconque des revendications 1 à 5, le coeur superparamagnétique étant recouvert d'un ou plusieurs polymères biocompatibles répondant à la formule suivante : 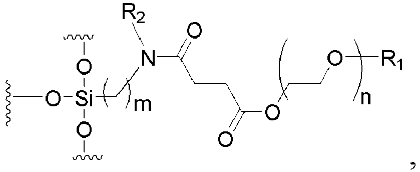 dans laquelle
R₁ est H, un alkyle en C₁ à C₆, un alcényle en C₂ à C₆, un alcynyle en C₂ à C₆, un cycloalkyle en C₃ à C₁₀, un hétérocycloalkyle en C₁ à C₁₀, un aryle, un hétéroaryle, un groupe carbonyle en C₁ à C₁₀, ou un groupe amine en C₁ à C₁₀ ;
R₂ est H, un alkyle en C₁ à C₆, un alcényle en C₂ à C₆, un alcynyle en C₂ à C₆, un cycloalkyle en C₃ à C₁₀, un hétérocycloalkyle en C₁ à C₁₀, un aryle, ou un hétéroaryle ;
m vaut de 1 à 10 ; et
n vaut de 5 à 1000.

11. Nanoparticules magnétiques biocompatibles pour une utilisation selon la revendication 10, lesquelles nanoparticules magnétiques biocompatibles ont chacune une granulométrie de 15 à 200 nm ; la maladie étant un rejet de greffe de rein ; le coeur superparamagnétique étant une nanoparticule d'oxyde de fer superparamagnétique ; le groupe polyéthylèneglycol ayant 10 à 200 motifs oxyéthylène ; le groupe silane contenant un alkylène en C₃ à C₁₀ ; le lieur étant un fragment carbonyle répondant à la formule suivante : 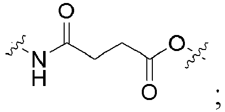 et l'image par résonance magnétique est une image par résonance magnétique pondérée T2 ou T2*.

12. Nanoparticules magnétiques biocompatibles pour une utilisation selon la revendication 10, R₁ étant H ; R₂ étant H, un alkyle en C₁ à C₆, un groupe carbonyle en C₁ à C₁₀, ou un groupe amine en C₁ à C₁₀ ; m vaut de 3 à 10 ; et n vaut de 10 à 200.

13. Nanoparticules magnétiques biocompatibles pour une utilisation selon l'une quelconque des revendications 1 à 10 et 12, lesquelles nanoparticules magnétiques biocompatibles ayant chacune une granulométrie de 10 à 1000 nm, de préférence lesquelles nanoparticules magnétiques biocompatibles ont chacune une granulométrie de 15 à 200 nm.
